# EUROPEAN PATENT SPECIFICATION

(11) **EP 4 415 667 B1**
(45) Date of publication and mention of the grant of the patent: **19.02.2025**
(21) Application number: 23707956.1
(22) Date of filing: 02.03.2023
(51) Int. Cl.: A61F 13/15, D04H 3/14, D04H 3/16, B41M 3/00

(54) **PRINTED NONWOVEN WEB WITH MICRODOT BOND PATTERN**
BEDRUCKTE VLIESSTOFFBAHN MIT MIKROPUNKTVERBINDUNGSMUSTER
BANDE NON TISSÉE IMPRIMÉE AVEC UN MOTIF DE LIAISON À MICROPOINTS

(30) Priority: 02.03.2022 EP 22159685
(43) Date of publication of application: 21.08.2024
(73) Proprietor: Fibertex Personal Care A/S, 9220 Aalborg (DK)
(72) Inventor: HAHNE, Florian, 38835 Lüttgenrode (DE); JACOBSEN, Jesper, 9270 Klarup (DK); UDENGAARD, Brian, 8520 Lystrup (DK)
(74) Representative: Frick, Robert
(86) International application number: PCT/EP2023/055260
(87) International publication number: WO 2023/166106

(56) References cited:
- EP-A1- 1 961 850
- EP-B1- 1 656 254
- WO-A1-2018/144293
- US-A1- 2007 049 889

## Description

The invention relates to nonwoven webs comprising a pattern of fusion-embossed bonding points on at least one of its surfaces and being printed on the same surface. The invention further relates to a method for making such nonwoven webs, and to uses for such nonwoven webs.

Hygiene industry uses nonwoven webs as materials for making products such as baby diapers or adult incontinence products on a large scale. Webs comprising or consisting of spunbonded nonwoven material and bonded by calender embossing have become industry standard for many applications. Examples comprise US 2014/0072767 A1. These webs not only have desirable product characteristics, they can also be economically produced at high line speeds. Depending on the type of application, the materials must be printed with texts, graphical patterns or images. Most typically, printing these materials is facilitated by flexographic ink printing, where the nonwoven web is fed to a flexographic printing unit at high translation speed and where ink is transferred from a print roll directly to the surface of the web. US 2007/049889 A1 discloses a method for printing on surfaces of nonwoven webs.

The image quality when ink-printing a calender-bonded nonwoven web comprising a pattern of fusion-embossed bonding points on its printed surface is limited, however. The reason is that the bonding points take on a higher color intensity and contrast than the remaining parts of the fabric, which makes the print appear pixelated and inhomogeneous.

The present invention aims to improve image quality for prints on calender-bonded nonwoven webs without detriment to production efficiency and economy.

Against this background the invention proposes a printed nonwoven web comprising a pattern of fusion-embossed bonding points, wherein the nonwoven web is ink-printed on the surface showing the patter of bonding points, wherein the pattern of bonding points fulfils the following conditions: (a) the number of bonding points on the surface is greater than 70/cm², preferably greater than 75/cm²; (b) the average area size of the individual bonding points is smaller 1.5 mm², preferably smaller than 1.35 mm²; and (c) the average distance between bonding points, from centre to centre, is smaller than 1.5 mm, preferably smaller than 1.3 mm. The bonding pattern is homogenous over the entire surface of the fabric.

The conditions are representative of using more and smaller bonding points than previously used in the art. The smaller bonding points lead to a surface with a more uniform appearance. This is especially advantageous for subsequent printing as it makes the print appear more uniform. The reason for this is that wherever a part of the printed design includes a bonding point, that area will appear to be slightly differently coloured than the unbonded printed surface due to the differences in smoothness and opacity. With the smaller bonding points and a higher density bonding pattern this becomes less pronounced, so that the printed design looks better.

In one embodiment, the bond area, defined as fraction of the total area of the surface that is occupied by the bonding points, is smaller than 18%, preferably smaller than 15%, more preferably than 12%, and yet more preferably smaller than 11%. The smaller the overall bond area, the smaller the overall area with slightly difference colouring. Hence, and overall smaller bond area can also contribute to better print quality.

The respective printed nonwoven webs can be produced by a method, wherein a nonwoven web is bonded by calender embossing to form a nonwoven web comprising a pattern of fusion-embossed bonding points fulfilling the above conditions on at least one of its surfaces, and wherein the nonwoven web is ink-printed on the surface showing the patter of bonding points.

Preferably, the ink printing is flexographic ink printing. In a flexographic ink printing unit, the nonwoven web is typically passed run across the surface of a print roll. The print roll comprises an exchangeable cylindrical sleeve folded around a core cylinder. The sleeve comprises a mirrored master of the required image as a positive 3D relief. Ink is transferred from an ink reservoir to the print roll from an anilox roll, which has surface cells for dosing the ink. Doctor blades scrape off excessive ink from outside the cells. The print roll comprises a mirrored master of the required image as a positive 3D relief.

Alternatively, the ink printing can also be ink jet printing. This printing method may not allow the same line speeds as flexographic printing, but is more flexible in terms of individualization or changes. Digital prints can be realized with ink jet printing.

The ink can be a water- or solvent-based ink and the surface can be dried after ink application. Alternatively, the ink can be a UV-curable ink that contains reactive monomers and/or oligomers, pigments and photoinitiators, in which the surface can be irradiated with UV light after the ink application. UV-curable inks can be advantageous from a standpoint of increased adhesion and reduced VOC emission.

The inventively configured bonding point pattern can be operated in a large range of print resolutions. Specifically, the contact areas on the print roller are typically dotted and the dots are arranged in lines. The line screen, as expressed in lines per cm, is representative for print resolution. In one embodiment, the line screen can be between 15 L/cm and 50 L/cm. Print resolutions of greater than about 30 L/cm could be used to obtain superior print quality.

In one embodiment, the nonwoven web is a spunbonded or spunmelt nonwoven material. Preferably, the material is formed from polyolefin fibers. Suitable polyolefins comprise, for example, polypropylene (PP), polyethylene (PP), or copolymers comprising propylene- or ethylene-based copolymers (co-PP; co-PE). A spunbonded nonwoven material may be combined with additional same or different spunbonded (S) layers and/or meltblown (M) layers to obtain multiple layer webs of SS, SMS or similar types.

During bonding the material is run across the surface of a calender roll having embossing projections on the surface that fulfil the conditions laid out above. In a preferred embodiment, bonding comprises ultrasonic bonding, where ultrasonic vibrations are introduced into the embossing projections and the fibers are fused together by ultrasonic fusion. Other embodiments use thermal bonding where the embossing projections are heated and the fibers are fused together by thermal fusion.

For optimum production efficiency, the method steps of nonwoven material formation by fiber laydown and bonding on the one hand and by printing on the nonwoven on the other hand are preferably carried out inline. Alternatively, for greater flexibility, an offline setting where a prefabricated nonwoven material is printed separately can be used.

In general, the line speed for nonwoven material formation and printing, be it together in an inline setting or separately in an offline setting, is preferably at least 100 meters per minute, more preferably greater than 150 meters per minute, and even more preferably greater than 200 meters per minute.

The printed nonwoven webs of the invention can be used in hygiene articles such as baby diapers, sanitary napkins, or adult incontinence products. Alternatively, the nonwoven webs can also be used in medical applications such as gowns, face masks, nursing pads, or bed sheets. Further, the nonwoven webs can be used in household products like wipes and cleaning towels.

By reducing the distance between the bonding points, the quality of the print gets very close to what can be achieved for other nonwoven types bonded without creating bonding points, like on air through carded, since the bond pattern becomes less pronounced. The bonding dots are so small that they do not interfere with the quality of the print. At the same time, production efficiency and product quality remains high. Specifically, the calander bonded nonwovens have better physical properties than nonwovens bonded by other methods like air through bonding.

Further details and advantages of the invention will become apparent from the figures and examples described in the following. The figures show:
- Figure 1:: a schematic setup of a production line for making printed nonwoven webs according to the invention;
- Figure 2:: an illustration showing the principle of flexographic ink printing;
- Figure 3:: a typical flexographic ink printing layout used in nonwoven printing;
- Figure 4:: a bonding pattern as used in the prior art; and
- Figure 5:: a bonding pattern according to the invention.

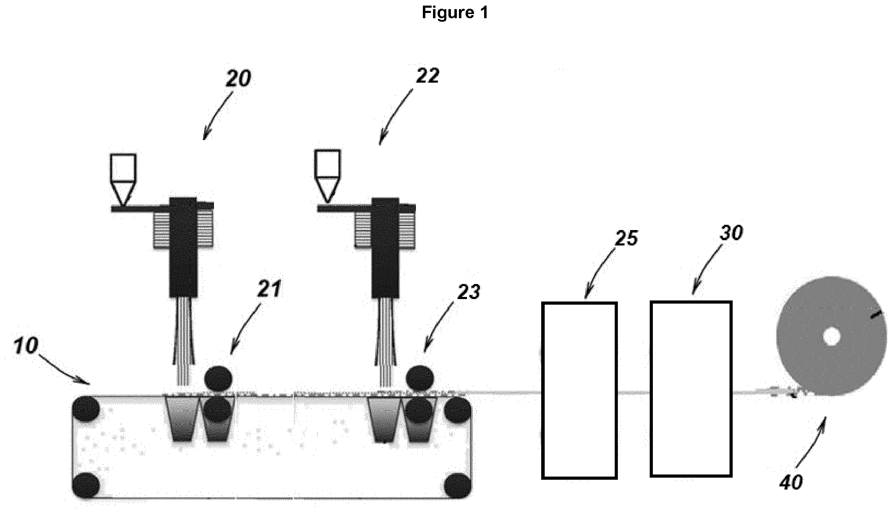

An exemplary machine setup for making printed nonwoven web 10 according to the invention is shown in Figure 1.

The setup comprises a conveyor belt and two spunbonding machines 20, 22 arranged in line on the conveyor belt. In each of the spunbonding machines, a molten thermoplastic polymer is extruded through the holes of a die. The extruded fiber strands are then quenched and drawn / stretched to form endless fibers, which are then laid onto the conveyor belt or, in case of the second laydown, on the first web that has been previously deposited thereon. Each of the spunbonding machines 20, 22 is followed by a pair of pre-compaction rollers 21, 23 for pre-compacting the respective webs.

The pre-compacted web is then bonded by calander-embossing in a bonding unit 25, where a fabric is run over the surface of a calander roll having a pattern of embossing projections on its surface. Thermal heat or ultrasonic energy is introduced into the projections by a source of thermal or ultrasonic energy and the web is fusion bonded at the respective locations to obtain a pattern of bonding points that corresponds to the pattern of projections on the calander roll surface.

The bonding is followed by a printing step in printing unit 30, which will be described in more detail in the following. The product web is finally collected on the product roll 40.

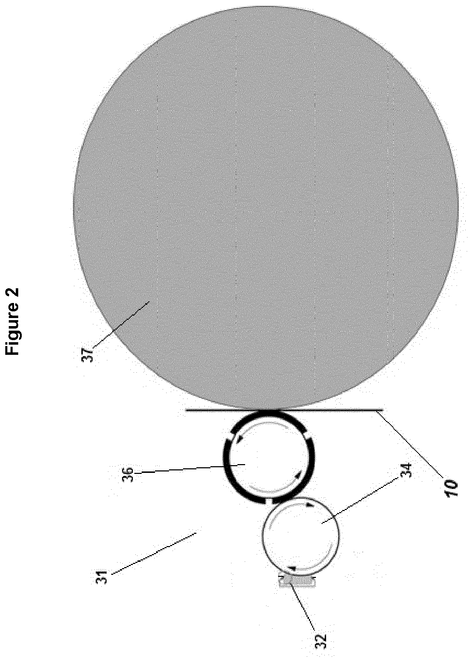

Figure 2 shows a flexographic printing unit 31 comprising a manifold for transferring ink onto the surface of an anilox roll 34. The manifold comprises an ink reservoir 32 and doctor blades acting as retaining and scrap blades. The surface of the anilox roll 34 comprises cells that enable dosing the ink to be transferred to the print roll 36. The surface of the print roll 36 comprises a positive mirrored master of the required image as a 3D relief. The print roll 36 transfers ink to the nonwoven web 10 while it is pressed against the print roll 36 by a counter-rotating impression drum 37.

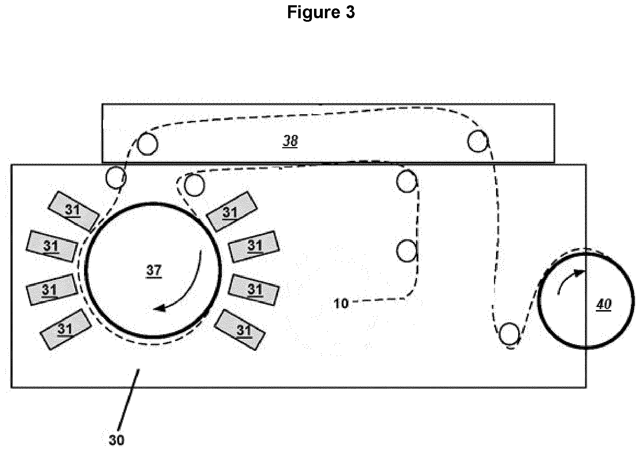

As shown in Figure 3, multiple units 31 comprising manifolds 33, anvil rolls 34 and print rolls 36 are arranged around a common impression drum 37 for multi-color printing in a printing unit 30. A drying or curing unit 38 follows inline.

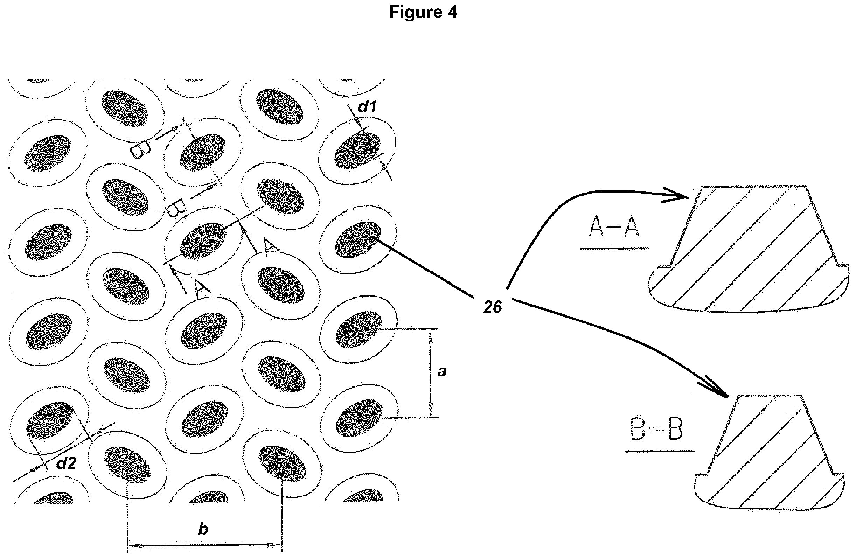

Figure 4 shows a pattern of embossing projections on the surface of a calander roll, that has been used for producing webs of the generic kind in the past. It comprises elliptically shaped embossing projections 26 that have different lengths d1 and widths d2. The bond area is approx. 0,25 mm². The distances a and b between the projections 26 are greater 1.5 and greater 2.5, respectively, and are such that the number of projections per area is approx. 50 and the bonding area, meaning the fraction of the total surface area occupied by the flat projection surfaces, is approx. 18%.

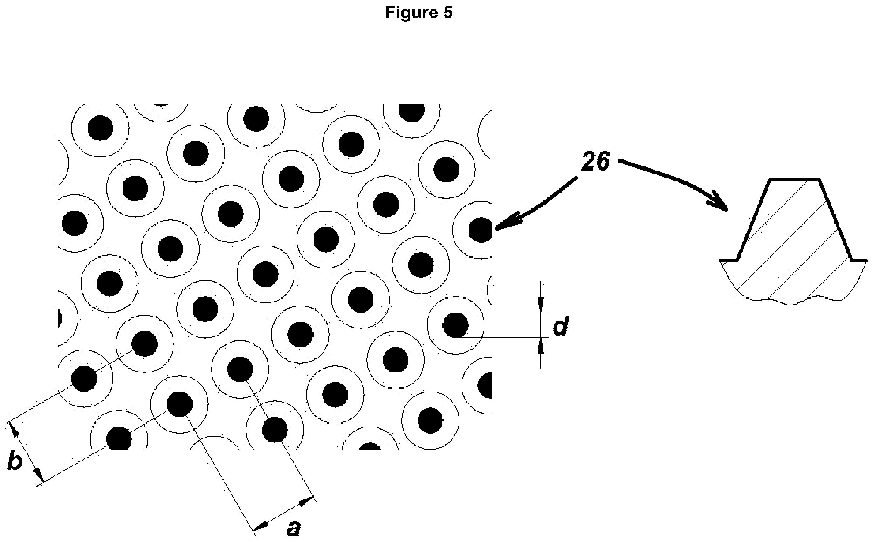

Figure 5 shows a pattern that is according to the invention. It is symmetrical and comprises circular embossing projections of a diameter d that is 0.4 mm. The bond area is hence approx. 0.125 mm². The pitch a (=b) between the bonding points is 1.12 mm. This results in a number of bonding points per area of 80/cm² and a total bonding area of 10%.

### Examples:

The examples compare the print quality on inventive microdot material and standard bonded nonwoven materials. All tests were carried out on the same 17 g/m² polypropylene three-layer spundbond (SSS) standard nonwoven material. The samples in the comparative tests were bonded using a bond pattern as in Figure 4. The samples in the inventive tests were bonded using a bond pattern as in Figure 5.

The samples all contained exactly the same print image and were printed, by flexographic ink printing, under exactly the same conditions (screen, sleeves, inks used, press, etc.).

The measurement procedure was as follows: In the first step, the exactly identical areas of the print samples to be compared are glued onto a white sheet of paper and scanned with a scanner (min. 300dpi resolution) as a PDF.The PDFs are then each opened in the software Adobe Photoshop 2023 and adjusted exactly to the area to be measured, before being converted to grayscale. Then, a gradation step is applied to separate the darker uneven areas from the even ones. The pixels are displayed in black after the gradation curve has been applied. For this purpose, a fixed sensitivity is set so that color tones with different brightness values can be measured. For the sensitivity setting, the L-value of the darkest shade of the area to be measured is determined using a measuring device (X-Rite Exact). The default sensitivity value (input value) with an L value of 75 is 48. If the L value deviates from +1, the sensitivity value decreases by 2. If the L value deviates from -1, the sensitivity value increases by 2.

Finally, with the help of the histogram, it can be read off how many deviating black pixels are present in the print area, and the values can be compared between the two types of materials.

In all pressure ranges with an L value below 85, the inventive materials have less deviation than the comparative materials. This has been found for four different pictures P1-P4 that are print designs used in the absorbent hygiene industry. The results are shown in Table 1 below.

**Table 1**

| Picture | P1 | P2 | P3 | P4 |
|---|---|---|---|---|
| Total printed pixels | 7.777.244 | 11.331.414 | 1.471.901 | 6.436.458 |
| Black pixels comparative sample | 89.520 | 290.296 | 18.090 | 124.389 |
| Ratio of black pixels in comparative sample | 1,2% | 2,6% | 1,2% | 1,9% |
| Black pixels inventive sample | 40.370 | 13.825 | 11.800 | 62.425 |
| Ratio of black pixels in inventive sample | 0,5% | 0,1% | 0,8% | 1,0% |
| Sensitivity | 48 | 72 | 58 | 48 |
| Total pixels | 10.164.610 | 11.331.414 | 8.076.819 | 8.958.048 |
| Darkest L Value | 75 | 63 | 70 | 75 |

It has been found for a number of different solid single color prints (rectangular). Corresponding results are given in Table 2 below.

**Table 2**

| Color | M100 | C100 | K100 | MY100 |
|---|---|---|---|---|
| Total printed pixels | 254.000 | 254.000 | 254.000 | 254.000 |
| Black pixels comparative sample | 9.390 | 14.821 | 6.327 | 5.880 |
| Ratio of black pixels in comparative sample | 3,7% | 5,8% | 2,6% | 2,3% |
| Black pixels inventive sample | 3.771 | 7.088 | 3.760 | 2.439 |
| Ratio of black pixels in inventive sample | 1,5% | 2,8% | 1,5% | 1.0% |
| Sensitivity | 44 | 46 | 48 | 48 |
| L Value | 77 | 76 | 75 | 75 |

**Table 2 (cntd.)**

| Color | YC100 | CM100 | P2593 100 |
|---|---|---|---|
| Total printed pixels | 254.000 | 254.000 | 254.000 |
| Black pixels comparative sample | 7.660 | 10.412 | 10.612 |
| Ratio of black pixels in comparative sample | 3,1% | 4,1% | 4,2% |
| Black pixels inventive sample | 4.670 | 4.579 | 3.020 |
| Ratio of black pixels in inventive sample | 1,8% | 1,8% | 1,2% |
| Sensitivity | 50 | 66 | 54 |
| L Value | 74 | 66 | 72 |

## Claims

1. A printed nonwoven web (10) comprising a pattern of fusion-embossed bonding points, wherein the nonwoven web (10) is ink-printed on the surface showing the pattern of bonding points,
**characterized in that**
the pattern of bonding points is homogenous over the entire surface of the fabric and fulfils the following conditions:
(a) the number of bonding points on the surface is greater than 70/cm², preferably greater than 75/cm²;
(b) the average area size of the individual bonding points is smaller than 1.5 mm², preferably smaller than 1.35 mm²; and
(c) the average distance between bonding points, from centre to centre, is smaller than 1.5 mm, preferably smaller than 1.3 mm.

2. The printed nonwoven web according to claim 1, wherein the bond area, defined as fraction of the total area of the surface that is occupied by the bonding points, is smaller than 18%, preferably smaller than 15%, more preferably than 12%, and yet more preferably smaller than 11%.

3. The printed nonwoven web according to any preceding claim, wherein the web (10) is a spunbonded or spunmelt nonwoven web.

4. A method for making a printed nonwoven web (10), wherein a nonwoven web is bonded by calender embossing to form a nonwoven web comprising a pattern of fusion-embossed bonding points, and wherein the nonwoven web is ink-printed on the surface showing the pattern of bonding points,
**characterized in that**
the pattern of bonding points is homogenous over the entire surface of the fabric and fulfils the following conditions:
(a) the number of bonding points on the surface is greater than 70/cm², preferably greater than 75/cm²;
(b) the average area size of the individual bonding points is smaller than 1.5 mm², preferably smaller than 1.35 mm²; and
(c) the average distance between bonding points, from centre to centre, is smaller than 1.5 mm, preferably smaller than 1.3 mm.

5. The method according to claim 4, wherein the bond area, defined as fraction of the total area of the surface that is occupied by the bonding points, is smaller than 18%, preferably smaller than 15%, more preferably than 12%, and yet more preferably smaller than 11%.

6. The method according to claim 4 or 5, wherein the ink printing is flexographic ink printing.

7. The method according to claim 6, wherein the line screen is between 15 L/cm and 50 L/cm.

8. The method according to claim 4 or 5, wherein the ink printing is ink jet printing.

9. The method according to any one of claims 4 to 8, wherein the web (10) is a spunbonded nonwoven web.

10. The method according to any one of claims 4 to 9, wherein the calender embossing comprises ultrasonic embossing.

11. Use of a printed nonwoven web (10) according to any preceding claim for the manufacture of hygiene articles, preferably for the manufacture of diapers.

## Patentansprüche

1. Bedruckter Vliesstoff (10), umfassend ein Muster von schmelzgeprägten Bindungspunkten, wobei der Vliesstoff (10) auf der Oberfläche mit Tinte bedruckt ist, die das Muster der Bindungspunkte zeigt,
**dadurch gekennzeichnet, dass**
das Muster der Bindungspunkte über die gesamte Oberfläche des Gewebes homogen ist und die folgenden Bedingungen erfüllt:
(a) die Anzahl der Bindungspunkte auf der Oberfläche größer als 70/cm², vorzugsweise größer als 75/cm² ist;
(b) die durchschnittliche Flächengröße der einzelnen Bindungspunkte kleiner als 1,5 mm², vorzugsweise kleiner als 1,35 mm² ist; und
(c) der durchschnittliche Abstand zwischen den Bindungspunkten, von Mitte zu Mitte, kleiner als 1,5 mm, vorzugsweise kleiner als 1,3 mm ist.

2. Bedruckter Vliesstoff nach Anspruch 1, wobei die Bindungsfläche, definiert als Bruchteil der Gesamtfläche der Oberfläche, die von den Bindungspunkten eingenommen wird, kleiner als 18 %, vorzugsweise kleiner als 15 %, noch bevorzugter kleiner als 12 % und noch bevorzugter kleiner als 11 % ist.

3. Bedruckter Vliesstoff nach einem der vorhergehenden Ansprüche, wobei der Stoff (10) ein Spinn- oder Spunmelt-Vliesstoff ist.

4. Verfahren zum Bilden eines bedruckten Vliesstoffs (10), wobei ein Vliesstoff durch Kalanderprägen gebunden wird, um einen Vliesstoff auszubilden, der ein Muster von schmelzgeprägten Bindungspunkten umfasst und wobei der Vliesstoff auf der Oberfläche mit Tinte bedruckt ist, die das Muster der Bindungspunkte zeigt,
**dadurch gekennzeichnet, dass**
das Muster der Bindungspunkte über die gesamte Oberfläche des Gewebes homogen ist und die folgenden Bedingungen erfüllt:
(a) die Anzahl der Bindungspunkte auf der Oberfläche größer als 70/cm², vorzugsweise größer als 75/cm² ist;
(b) die durchschnittliche Flächengröße der einzelnen Bindungspunkte kleiner als 1,5 mm², vorzugsweise kleiner als 1,35 mm² ist; und
(c) der durchschnittliche Abstand zwischen den Bindungspunkten, von Mitte zu Mitte, kleiner als 1,5 mm, vorzugsweise kleiner als 1,3 mm ist.

5. Verfahren nach Anspruch 4, wobei die Bindungsfläche, definiert als Bruchteil der Gesamtfläche der Oberfläche, die von den Bindungspunkten eingenommen wird, kleiner als 18 %, vorzugsweise kleiner als 15 %, noch bevorzugter kleiner als 12 % und noch bevorzugter kleiner als 11 % ist.

6. Verfahren nach Anspruch 4 oder 5, wobei der Tintendruck mit Flexodruckfarbe erfolgt.

7. Verfahren nach Anspruch 6, wobei das Linienraster zwischen 15 L/cm und 50 L/cm liegt.

8. Verfahren nach Anspruch 4 oder 5, wobei der Tintendruck ein Tintenstrahldruck ist.

9. Verfahren nach einem der Ansprüche 4 bis 8, wobei der Stoff (10) ein Spinnvliesstoff ist.

10. Verfahren nach einem der Ansprüche 4 bis 9, wobei die Kalanderprägung das Ultraschall-Prägen umfasst.

11. Verwendung eines bedruckten Vliesstoffs (10) nach einem der vorhergehenden Ansprüche zur Herstellung von Hygieneartikeln, vorzugsweise zur Herstellung von Windeln.

## Revendications

1. Bande de non tissé imprimée (10) comprenant un motif de points de liage gaufrés par fusion, la bande de non tissé (10) étant imprimée à l'encre sur la surface montrant le motif de points de liage,
**caractérisée en ce que**
le motif de points de liage est homogène sur toute la surface du tissu et satisfait aux conditions suivantes :
(a) le nombre de points de liage sur la surface est supérieur à 70/cm², de préférence supérieur à 75/cm² ;
(b) la dimension de superficie moyenne des points de liage individuels est inférieure à 1,5 mm², de préférence inférieure à 1,35 mm² ; et
(c) la distance moyenne entre des points de liage, de centre à centre, est inférieure à 1,5 mm, de préférence inférieure à 1,3 mm.

2. La bande de non tissé imprimée selon la revendication 1, dans laquelle la superficie de liage, définie comme une fraction de la superficie totale de la surface occupée par les points de liage, est inférieure à 18 %, de préférence inférieure à 15 %, de préférence encore à 12 %, et de préférence encore plus, inférieure à 11 %.

3. La bande de non tissé imprimée selon une revendication précédente quelconque, dans laquelle la bande (10) est une bande de non tissé filé-lié ou filé-fondu.

4. Procédé de fabrication d'une bande de non tissé imprimée (10), dans lequel une bande de non tissé est liée par gaufrage à la calandre afin de former une bande de non tissé comprenant un motif de points de liage gaufrés par fusion, et dans lequel la bande de non tissé est imprimée à l'encre sur la surface montrant le motif de points de liage,
**caractérisé en ce que**
le motif de points de liage est homogène sur toute la surface du tissu et satisfait aux conditions suivantes :
(a) le nombre de points de liage sur la surface est supérieur à 70/cm², de préférence supérieur à 75/cm² ;
(b) la dimension de superficie moyenne des points de liage individuels est inférieure à 1,5 mm², de préférence inférieure à 1,35 mm² ; et
(c) la distance moyenne entre des points de liage, de centre à centre, est inférieure à 1,5 mm, de préférence inférieure à 1,3 mm.

5. Le procédé selon la revendication 4, dans lequel la superficie de liage, définie comme une fraction de la superficie totale de la surface occupée par les points de liage, est inférieure à 18 %, de préférence inférieure à 15 %, de préférence encore à 12 %, et de préférence encore plus, inférieure à 11 %.

6. Le procédé selon les revendications 4 ou 5, dans lequel l'impression à l'encre est une impression à l'encre flexographique.

7. Le procédé selon la revendication 6, dans lequel la trame à lignes est entre 15 L/cm et 50 L/cm.

8. Le procédé selon les revendications 4 ou 5, dans lequel l'impression à l'encre est une impression au jet d'encre.

9. Le procédé selon l'une quelconque des revendications 4 à 8, dans lequel la bande (10) est une bande de non tissé filé-lié.

10. Le procédé selon l'une quelconque des revendications 4 à 9, dans lequel le gaufrage à la calandre comprend un gaufrage à ultrasons.

11. Utilisation d'une bande de non tissé imprimée (10) selon une revendication précédente quelconque pour la confection d'articles d'hygiène, de préférence pour la confection de couches.
